# EUROPEAN PATENT APPLICATION

(11) **EP 2 216 040 A1**
(43) Date of publication of application: **11.08.2010**
(21) Application number: 07845763.7
(22) Date of filing: 30.11.2007
(51) Int. Cl.: A61K 36/725, A61K 36/484, A61K 36/258, A61P 25/24

(54) **PHARMACEUTICAL COMPOSITIONS WITH A MECHANISM OF MULTI-TARGET RECEPTOR RETROACTION FOR TREATING DEPRESSION**

(71) Applicant: Chi, Yu-Fen, Taiwan (CN); Zhang, Zuoguang, Beijing 100000 (CN)
(72) Inventor: ZHANG, Zuoguang, Beijing 100000 (CN)
(74) Representative: Adler, Peter
(86) International application number: PCT/CN2007/003400
(87) International publication number: WO 2009/070924

(57) **Abstract**

Oral pharmaceutical compositions or functional foods with a mechanism of multi-target receptor retroaction for treating depression comprising ginseng saponin (Rg1 + Rb1), glycyrrhizic acid and jujuba cAMP. Experiments demonstrate that as compared with the preferred drug for treating depression Paroxetine in the art, the present invention has significant anti-depression efficacy.

## Description

The present invention relates to a multi-target, post-receptor mechanism-based pharmaceutical composition manufactured from the raw materials of ginsenoside Rg1 and Rb1, glycyrrhizic acid and jujuba cyclic adenosine monophosphate (jujuba cAMP) for treating depression. In particular, the present invention relates to an oral pharmaceutical or a health food for treating depression that has definite functions and components, obvious curative effects and high safety for long-term usage, and that is devoid of side effects such as severe emesis.

Depression is a common disease. According to statistics, about 25% females in the global population had been experiencing depression in their lives, and about 10% males had been experiencing depression (referring to *Modern Psychology* written by Ch'un-Hsing Chang). World Health Organization (WHO) published, "The incidence of depression in the world is about 11%. At present, about 340 million psychological depressed patients are in the world, and the number is increasing. It is found in the investigation that the depression will increase to be the number two common disease in the world from now on to 20 years later."

At present, the main anti-depression pharmaceuticals are Prozac, Paxil and Zoloft, etc., which belong to selective serotonin reuptake inhibitor (SSRI), serotonin-norepinephrine reuptake inhibitor (SNRI), and norepinephrine and dopamine reuptake inhibitor (NDRI), inhibiting the uptake of neurotransmitters such as 5-hydroxytryptamine (5-HT), norepinephrine (NE) and dopamine (DA). The mechanism by which these anti-depression pharmaceuticals function is by increasing the content of the human neurotransmitter 5-HT so as to reduce and alleviate the symptoms of depression.

However, the marketed anti-depression pharmaceuticals have various side effects with different severities, such as increased suicide rate, headache, giddiness, vertigo, insomnolence, hypersomnia, tinnitus, thirsty, apocleisis, orexis, increased body weight, increased blood pressure, stomach upset, regurgitation nausea, emesis, dyspepsia, diarrhea, constipation, leg pain, skin rash, dither, convulsions, hyperhidrosis, edema, sexual appetite, and impotence, etc. In recent years, the depression pharmaceuticals, such as Prozac, etc., had become a serious social problem. In 2004, the Food and Drug Administration (FDA) of the United States further mandated the pharmaceutical companies to clearly state the side effects and cautions in the instructions of 32 major anti-depression pharmaceuticals in the market, and emphasized to physicians and nurses that these pharmaceuticals might increase children's and adolescents' suicide rates. Among the 32 anti-depressants, Paxil was even found to be harmful in 1996, and has been recalled continually from the market since 2001. In June 2004, the New York State Attorney General accused GlaxoSmithKline Company of the Great Britain of beguilingly concealing the research report of the linkage between Paxil and "increased risk of suicidal behavior and tendencies in adolescents." In light of the current situation, the search for a new generation of pharmaceuticals with less side effects and more pronounced/potent anti-depression qualities has become the center of attention of the entire pharmaceutical world.

In recent years, scientists in pharmacology have made a new breakthrough in the research of pathogenic mechanisms of depression, they found that in addition to inhibition of the reuptake of neurotransmitters such as 5-HT, NE and DA as a strategy for treating depression, regulation of the post-receptor mechanism could also be adopted to treat depression. Furthermore, since the post-receptor mechanism regulating pharmaceutical, Rolipram, appeared, the post-receptor mechanism has become the research hotspot for treating depression in pharmacology. Rolipram is an inhibitor of phosphodiesterase 4 (PDE4) and has been shown in clinical trials to have obvious anti-depression function; however, because Rolipram causes severe emesis, further research on Rolipram was compelled to stop. Despite of this setback, Rolipram has opened up a brand-new thought of "post-receptor mechanism for anti-depression pharmaceutical".

It is therefore attempted by the applicant to deal with the above situation encountered in the prior art.

In order to overcome the insufficiency of the present technology, the purpose of the present invention is to provide a multi-target and post-receptor mechanism-based oral pharmaceutical or a health food, as manufactured from the raw materials of ginsenoside Rg1 and Rb1, glycyrrhizic acid and jujuba cAMP, for treating depression. In particular, new technical schemes resulting in definite functions and components, obvious curative effect and high safety for the long-term usage but without side effects such as severe emesis, etc., are provided.

The resolving scheme of the pharmaceutical of the present invention is the result endeavored by the inventor in accordance with the pathological and pharmacological theories of modern medicine for treating depression. In particular, the present invention combines recent research progress on anti-depression pharmaceuticals and the post-receptor mechanisms by harnessing the stimulatory effect of ginsenoside on adenylate cyclase (AC) and the strong inhibitory effect of glycyrrhizic acid (and glycyrrhetinic acid) on cAMP phosphodiesterase (CAPD). The anti-depression function of the present invention has been proven in plenty of animal experiments. Ginsenoside and glycyrrhizic acid (and glycyrrhetinic acid) when paired act synergistically to further increase the usage and activity of cAMP. The resulting concentration and activity of cAMP can (1) increase the synthesis and release of the neurotransmitter such as norepinephrine, etc., (2) increase the expression of the brain-derived neurotrophic factor (BDNF), and (3) inhibit the hyperactivity of the hypothalamic-pituitary-adrenal (HPA) axis and the secretion of glucocorticoid, so as to achieve the significant anti-depression function. In addition, jujuba cAMP can also increase the level of cAMP in human so as to induce the anti-depression function. A trace amount of jujuba cAMP (about one ten thousand) of jujuba is extracted and purified as the jujuba extract having 1% of jujuba cAMP to proceed the anti-experimental depression function in the animal experiments. The result shows that the jujuba extract containing 1% of jujuba cAMP has significant anti-experimental depression function. However, the regular jujuba extract (e.g. jujuba extracted in the water by heating, but the concentration of jujuba cAMP therein is not further increased), although containing a trace amount of jujuba cAMP, does not have obvious anti-experimental depression function. In order to further increase the anti-depression effect of the present invention in which jujuba cAMP, ginsenoside and glycyrrhizic acid are further paired and acted collectively. Ginseng, liquorice and jujuba are common-used pharmaceutical materials and food in Chinese medicine and have been used in dietary nourishing medicinal meals for several thousand years. In the thousands of years' history of medicinal and dietary usage, the safety of ginseng, liquorice and jujuba taken in pair and together has been sufficiently proven. The inventor's research and experimental results show that if these three pharmaceutical materials are only normally decocted and extracted individually, the extract does not have significant anti-depression effect as compared to the extract with the present anti-depression pharmaceutical for treating depression. This is because in the present invention the concentrations of effective components (such as ginsenoside Rg1 and Rb1, and jujuba cAMP, etc.) of the extract are further increased by purification (such as chromatography), and the raw materials (such as glycyrrhizic acid and glycyrrhetinic acid, etc.) are added to the extract for preparing the pharmaceutical for treating depression The animal experiments demonstrate that the pharmaceutical thus generated has significantly superior anti-depression function compared with previous anti-depression pharmaceuticals. The debris from those three pharmaceutical materials after extraction, chromatography and purification is also collected and examined by high performance liquid chromatography (HPLC). Although the debris contains a trace amount of ginsenoside Rg1 and Rb1, glycyrrhizic acid and jujuba cAMP, the debris does not have significant anti-depression function as tested by the animal experiments. Importantly, taking ginseng, liquorice and jujuba would not generate side effects such as severe emesis, etc. Therefore, the inventor submits the oral pharmaceutical or health food manufactured from the raw materials including ginsenoside Rg1 and Rb1, glycyrrhizic acid and jujuba cAMP, with multi-target and post-receptor mechanism for treating depression. In particular, in addition to extraction by heating in the solvent (such as water and ethanol, etc.), the extract is further purified to increase the concentrations of raw materials (ginsenoside Rg1 and Rb1, and jujuba cAMP), and glycyrrhizic acid and glycyrrhetinic acid are added therein. New technical scheme is prepared to have definite functions and components, obvious curative effect, high safety for long-term usage, and without inducing side effects such as severe emesis, etc., for overcoming the insufficiency of the currently available anti-depressants.

The comparisons of the anti-depression pharmaceutical target and the mechanism of the pharmaceutical composition of the present invention and Rolipram are provided as follows.

| Drug name | Rolipram | Pharmaceutical composition of the present invention |
|---|---|---|
| Drug type | Post-receptor mechanism regulating anti-depression pharmaceutical (chemical pharmaceutical) | Post-receptor mechanism regulating anti-depression pharmaceutical (compound pharmaceutical) |
| Drug target | Single target (CAPD) | Multi-target (CAPD, CA and ATP, etc.) |
| Mechanism | 1. Triggering cAMP-dependent tyrosine hydroxylase for increasing norepinephrine synthesis; 2. phosphorylating synapsin and increasing the release of norepinephrine; and 3. inhibiting phosphodiesterase, decreasing cAMP degradation, and increasing the usage of cAMP for generating downstream function. | 1. Triggering cAMP-dependent tyrosine hydroxylase for increasing norepinephrine synthesis; 2. phosphorylating synapsin and increasing the release of norepinephrine; 3. inhibiting phosphodiesterase, decreasing cAMP degradation, and increasing the usage of cAMP for developing the function; 4. triggering AC, increasing the concentration and activity of cAMP; 5. increasing ATP for promoting the synthesis of cAMP; 6. promoting benzenepropionic acid by blood-brain barrier, promoting the synthesis of NE and DA, and down-regulating HPA axis; and 7. up-regulating the expression of BDNF and neurotrophin (NT-3) directly in the brain cells for anti-stress reaction. |
| Dosage reaction | Inducing side effects such as severe emesis, etc. | High safety and low side effects for long-term usage, and preventing and improving retrograde nervous disorders and anti-senescence. |

The differences between the pharmaceutical of the present invention and the anti-depression pharmaceutical with the post-receptor mechanism (Rolipram) lie in that the anti-depression reaction is achieved by multi-target and post-receptor mechanism, and the side effect induced by Rolipram (such as severe emesis) is avoided.

The conversion rate of glycyrrhizic acid to glycyrrhetinic acid in the human body is almost 100% and glycyrrhetinic acid, owing to its higher liposolubility than glycyrrhizic acid, can enter into the brain through the blood-brain barrier. Therefore, the inhibition of glycyrrhizic acid on CAPD is proceeded by transformation of glycyrrhizic acid to glycyrrhetinic acid in the body. Accordingly, the glycyrrhizic acid or glycyrrhetinic acid can be the raw material to manufacture the pharmaceutical composition of the present invention.

In accordance with one aspect of the present invention, a pharmaceutical composition with a multi-target post-receptor mechanism for treating depression is provided. The pharmaceutical composition includes: ginsenoside Rg1 and Rb1, a glycyrrhizically related acid being one selected from a group consisting of glycyrrhizic acid, glycyrrhetinic acid and a combination thereof, and jujuba cAMP.

Preferably, the pharmaceutical composition includes 2 ∼ 26 parts by weight of ginsenoside, 3 ∼ 48 parts by weight of the glycyrrhizically related acid and 0.002 - 0.5 parts by weight of jujuba cAMP.

Preferably, the pharmaceutical composition includes 4 ∼ 13 parts by weight of ginsenoside, 5 ∼ 16 parts by weight of the glycyrrhizically related acid and 0.01 ∼ 0.1 parts by weight of jujuba cAMP.

Preferably, ginsenoside is extracted from ginseng, the glycyrrhizically related acid is extracted from liquorice, and jujuba cAMP is extracted from jujuba.

Preferably, jujuba is extracted for obtaining a first extract having a first jujuba cAMP concentration, the first extract is further extracted for obtaining a second extract having a second jujuba cAMP concentration, the second jujuba cAMP concentration is higher than the first jujuba cAMP concentration, and the second extract is a raw material in the pharmaceutical composition.

In accordance with another aspect of the present invention, a multi-target post-receptor pharmaceutical composition for treating depression is provided. The pharmaceutical composition includes: ginsenoside; and a glycyrrhizically related acid being one selected from a group consisting of glycyrrhizic acid, glycyrrhetinic acid and a combination thereof.

Preferably, ginsenoside includes Rg1 and Rb1.

Preferably, the pharmaceutical composition includes 2 ∼ 26 parts by weight of ginsenoside and 3 ∼ 48 parts by weight of the glycyrrhizically related acid.

Preferably, the pharmaceutical composition includes 4 ∼ 13 parts by weight of ginsenoside and 5 ∼ 16 parts by weight of the glycyrrhizically related acid.

Preferably, the ginsenoside is purified from a ginseng, and the glycyrrhizically related acid is purified from a liquorice.

Preferably, the pharmaceutical composition includes at least one of a pharmacologically acceptable carrier and an additive.

Preferably, the pharmaceutical composition has a dosage form being one selected from a group consisting of a tablet, a capsule, a powder, a pill, a dust, a solution, a microcapsule, a suspension, an emulsion, a particle, a dropping pill and a roll.

Preferably, the pharmaceutical composition is manufactured as one of a health food and a nutrient supplement.

In accordance with another aspect of the present invention, a preparation method of a pharmaceutical composition with a multi-target post-receptor mechanism for treating depression is provided. The pharmaceutical composition includes jujuba cAMP as a raw material. The preparation method includes steps of: (a) extracting a jujuba for obtaining a first extract having a first jujuba cAMP concentration; and (b) purifying the first extract for obtaining a second extract having a second jujuba cAMP concentration, wherein the second jujuba cAMP concentration is higher than the first jujuba cAMP concentration.

Preferably, the step (b) is processed by chromatographing the first extract with a macroporous resin bound with an aldehyde group.

Preferably, the macroporous resin is OU-2.

Preferably, the first extract is further chromatographed with an ME-2 macroporous resin bound with the aldehyde group.

The oral pharmaceutical for treating depression described in the specification and claims of the present invention is the core content of the purpose of the present invention. After the present invention is published, one skilled in the art can proceed the normal increase/decrease or substitute for other effective components of the herbal medicine (such as onjisaponin, saikosaponin and liquorice coumarin, etc.) having identical effects/functions to the above-mentioned pharmaceutical in accordance with the theory of Chinese medicine or related theory of modern pharmacology. The substitutions of this normal increase/decrease, the herbal medicine having similar mechanism, other identical CAPD inhibitor, AC initiator, or corresponding effective components belong to the normal technical activities of one skilled in the art. Therefore, the substitutions are in the protecting scope of the present invention.

The above objectives and advantages of the present invention will become more readily apparent to those ordinarily skilled in the art after reviewing the following detailed descriptions and accompanying drawings, in which:

Fig. 1 is a flowchart showing a preparation method of a pharmaceutical composition in accordance with a first preferred embodiment of the present invention;

Fig. 2 is a flowchart showing a preparation method of a pharmaceutical composition in accordance with a second preferred embodiment of the present invention;

Fig. 3 is a flowchart showing a preparation method of a pharmaceutical composition in accordance with a third preferred embodiment of the present invention;

Fig. 4 is a flowchart showing a preparation method of a pharmaceutical composition in accordance with a fourth preferred embodiment of the present invention;

Fig. 5 is a flowchart showing a preparation method of a pharmaceutical composition in accordance with a fifth preferred embodiment of the present invention; and

Fig. 6 is a flowchart showing a preparation method of a pharmaceutical composition in accordance with a sixth preferred embodiment of the present invention.

The present invention will now be described more specifically with reference to the following Embodiments. It is to be noted that the following descriptions of preferred Embodiments of this invention are presented herein for purpose of illustration and description only; it is not intended to be exhaustive or to be limited to the precise form disclosed.

In order to accomplish the purpose of the present invention, the technical schemes of the present invention are particularly provided as follows.

A pharmaceutical composition with multi-target and post-receptor mechanism for treating depression is disclosed in the present invention, and the pharmaceutical composition is manufactured by including the raw materials of ginsenoside Rg1 and Rb1, and glycyrrhizic acid (or glycyrrhetinic acid).

### Example 1:

The pharmaceutical composition with multi-target and post-receptor mechanism of the present invention for treating depression is manufactured by including the raw materials of ginsenoside Rg1 and Rb1, and glycyrrhizic acid (or glycyrrhetinic acid).

### Example 2:

The pharmaceutical composition of the present invention is manufactured from the raw materials having a total of 2 - 26 parts by weight of ginsenoside Rg1 and Rb1, and 3 ∼ 48 parts by weight of glycyrrhizic acid (or glycyrrhetinic acid).

### Example 3:

The pharmaceutical composition of the present invention is manufactured by including the raw materials having a total of 4 - 13 parts by weight of ginsenoside Rg1 and Rb1, and 5 ∼ 16 parts by weight of glycyrrhizic acid (or glycyrrhetinic acid).

### Example 4:

The pharmaceutical composition with multi-target and post-receptor mechanism for treating depression of the present invention is manufactured by including the raw materials of ginsenoside Rg1 and Rb1, glycyrrhizic acid (or glycyrrhetinic acid) and jujuba cAMP.

### Example 5:

The pharmaceutical composition of the present invention is manufactured by including the raw materials having a total of 2 - 26 parts by weight of ginsenoside Rg1 and Rb1, 3 ∼ 48 parts by weight of glycyrrhizic acid (or glycyrrhetinic acid) and 0.002 - 0.5 parts by weight of jujuba cAMP.

### Example 6:

The pharmaceutical composition of the present invention is manufactured by including the raw materials having a total of 4 - 13 parts by weight of ginsenoside Rg1 and Rb1, 5 ∼ 16 parts by weight of glycyrrhizic acid (or glycyrrhetinic acid) and 0.01 - 0.1 parts by weight of jujuba cAMP.

### Example 7:

The pharmaceutical composition of the present invention can include the pharmacologically acceptable carriers or additives. The pharmaceutical composition can be manufactured as a dosage form, and the dosage form is selected from one of a tablet, a capsule, a powder, a pill, a dust, a solution, a microcapsule, a suspension, an emulsion, a particle, a dropping pill, a roll and a pharmacologically oral pharmaceutical dosage form.

### Example 8:

The pharmaceutical composition of the present invention can be manufactured as pharmaceuticals, health food and nutrient supplements for treating depression.

In order to accomplish the purpose of the present invention, the preparation methods of the pharmaceutical composition are described as follows.

### Method 1:

The pharmaceutical composition with multi-target and post-receptor mechanism of the present invention for treating depression is manufactured from one extract having ginsenoside Rg1 and Rb1 extracted from ginseng, and another extract having glycyrrhizic acid extracted from liquorice as the raw materials. In addition, the aforementioned pharmaceutical composition is manufactured by directly using the prepared raw materials having ginsenoside Rg1 and Rb1, and glycyrrhizic acid (or glycyrrhetinic acid).

### Method 2:

The pharmaceutical composition of the present invention is manufactured from the raw materials having a total of 2 - 26 parts by weight of ginsenoside Rg1 and Rb1, and 3 ∼ 48 parts by weight of glycyrrhizic acid (or glycyrrhetinic acid).

### Method 3:

The pharmaceutical composition of the present invention is manufactured by including the raw materials having a total of 4 - 13 parts by weight of ginsenoside Rg1 and Rb1, and 5 ∼ 16 parts by weight of glycyrrhizic acid (or glycyrrhetinic acid).

### Method 4:

The pharmaceutical composition with multi-target and post-receptor mechanism of the present invention for treating depression is manufactured from one extract having ginsenoside Rg1 and Rb1 extracted from ginseng, another extract having glycyrrhizic acid extracted from liquorice, and the other extract having jujuba cAMP extracted from jujuba as the raw materials. In addition, the aforementioned pharmaceutical composition is manufactured by directly using the prepared raw materials having ginsenoside Rg1 and Rb1, glycyrrhizic acid (or glycyrrhetinic acid) and jujuba cAMP.

### Method 5:

The pharmaceutical composition of the present invention is manufactured from the raw materials having a total of 2 - 26 parts by weight of ginsenoside Rg1 and Rb1, 3 ∼ 48 parts by weight of glycyrrhizic acid (or glycyrrhetinic acid) and 0.002 - 0.5 parts by weight of jujuba cAMP.

### Method 6:

The pharmaceutical composition of the present invention is manufactured from the raw materials having a total of 4 - 13 parts by weight of ginsenoside Rg1 and Rb1, 5 ∼ 16 parts by weight of glycyrrhizic acid (or glycyrrhetinic acid) and 0.01 ∼ 0.1 parts by weight of jujuba cAMP.

### Method 7:

The pharmaceutical composition of the present invention can include the pharmacologically acceptable carriers or additives. The pharmaceutical composition can be manufactured as a dosage form, and the dosage form is selected from one of a tablet, a capsule, a powder, a pill, a dust, a solution, a microcapsule, a suspension, an emulsion, a particle, a dropping pill, a roll and a pharmacologically oral pharmaceutical dosage form.

### Method 8:

The pharmaceutical composition of the present invention can be manufactured from the raw materials described in the present invention in accordance with the method of the Good Manufacturing Practice (GMP) about the health food.

### THE PREFERRED EMBODIMENT

The present invention is further illustrated as follows by combining the figures and the preferred embodiments.

### Embodiment 1:

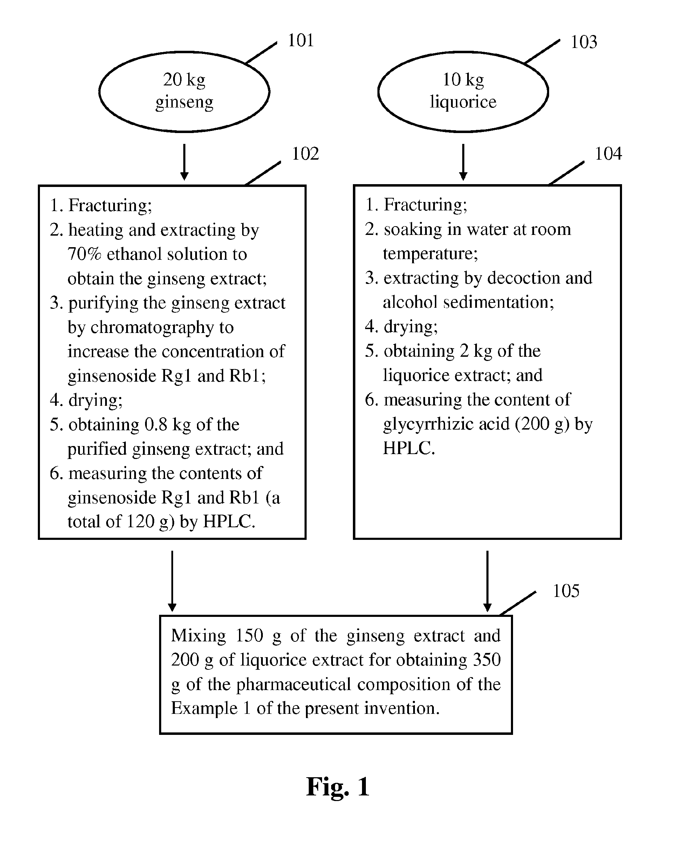

Please refer to Fig. 1, which is the flowchart showing a preparation method of a pharmaceutical composition in accordance with a first preferred embodiment of the present invention. In Fig. 1, after 20 kg of ginseng (step 101) is fractured, the fractured ginseng is heated to be extracted by 70% concentration of the ethanol solution. The extracted ginseng is separated by column chromatography, purified and dried, and 0.8 kg of the ginseng extract having 120 g of ginsenoside Rg1 and Rb1 is obtained (step 102). Then, after 10 kg of liquorice (step 103) is fractured, the fractured liquorice is soaked at room temperature for 12 hours. The soaked liquorice is extracted by decoction and alcohol sedimentation, concentrated and dried, and 2 kg of the liquorice extract having 200 g of glycyrrhizic acid is obtained (step 104). Afterwards, 150 g of the obtained ginseng extract and 200 g of the obtained liquorice extract are pulverized and mixed, and 350 g of the pharmaceutical composition (containing 22.5 g of ginsenoside Rg1 and Rb1, and 20 g of glycyrrhizic acid) of the Example 1 of the present invention is obtained (step 105).

### Embodiment 2:

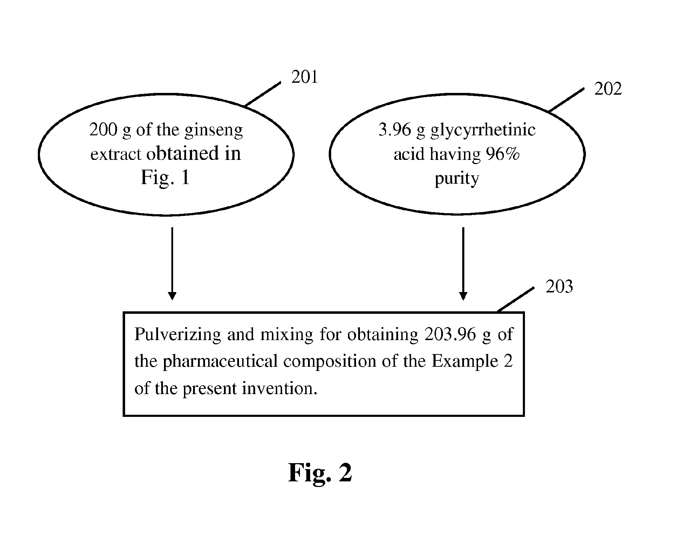

Please refer to Fig. 2, which is the flowchart showing a preparation method of a pharmaceutical composition in accordance with a second preferred embodiment of the present invention. In Fig. 2, after the prepared 3.96 g of glycyrrhetinic acid having 96% purity (step 202) and 200 g of the obtained ginseng extract in the Embodiment 1 (step 201) are pulverized and mixed, 203.96 g of the pharmaceutical composition (containing 30 g of ginsenoside Rg1 and Rb1, and 3.8 g of glycyrrhetinic acid) of the Example 2 of the present invention is obtained (step 203).

### Embodiment 3:

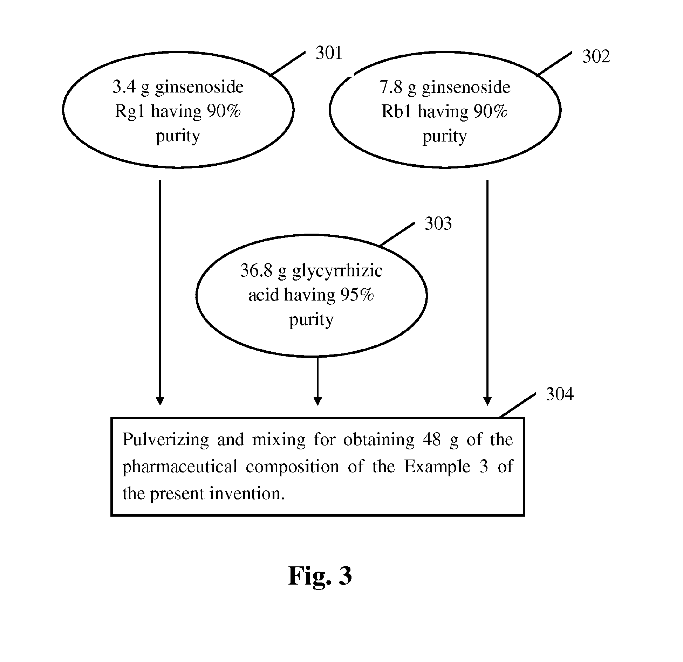

Please refer to Fig. 3, which is the flowchart showing a preparation method of a pharmaceutical composition in accordance with a third preferred embodiment of the present invention. In Fig. 3, after 3.4 g of the prepared ginsenoside Rg1 having 90% purity (step 301), 7.8 g of the prepared ginsenoside Rb1 having 90% purity (step 302) and 36.8 g of glycyrrhizic acid having 90% purity (step 303) are pulverized and mixed, 48 g of the pharmaceutical composition (containing 10 g of ginsenoside Rg1 and Rb1, and 35 g of glycyrrhizic acid) of the Example 3 of the present invention is obtained (step 304).

### Embodiment 4:

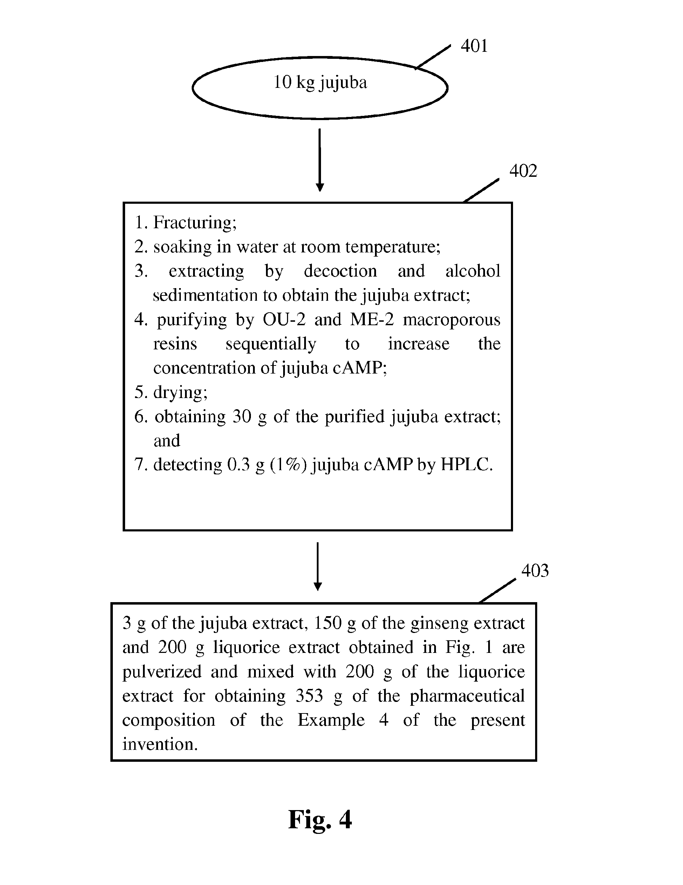

Please refer to Fig. 4, which is the flowchart showing a preparation method of a pharmaceutical composition in accordance with a fourth preferred embodiment of the present invention. In Fig. 4, 10 kg of jujuba (step 401) is fractured and soaked in water at room temperature. Then, the soaked jujuba is extracted by decoction and alcohol sedimentation for obtaining the jujuba extract, which is further absorbed and separated by the OU-2 and ME-2 macroporous resins sequentially, and dried. Thirty (30) g of the jujuba extract containing 0.3 g of jujuba cAMP is obtained to be the raw material for preparing the pharmaceutical of the present invention (step 402).

Afterwards, after 150 g of the ginseng extract and 200 g of the liquorice extract obtained in the Embodiment 1 are pulverized and mixed with 3 g of the aforementioned jujuba extract, 353 g of the pharmaceutical composition (containing 22.5 g of ginsenoside Rg1 and Rb1, 20 g of glycyrrhizic acid and 0.03 g of jujuba cAMP) of the Example of the present invention is obtained (step 403).

### Embodiment 5:

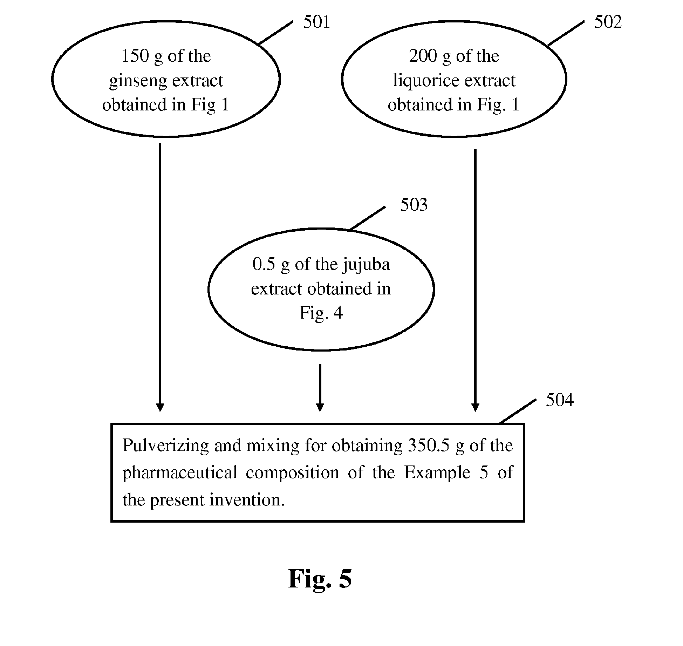

Please refer to Fig. 5, which is the flowchart showing a preparation method of a pharmaceutical composition in accordance with a fifth preferred embodiment of the present invention. In Fig. 5, after 150 g of the ginseng extract (step 501) and 200 g of the liquorice extract (step 502) obtained in the Embodiment 1 respectively are pulverized and mixed with 0.5 g of the jujuba extract obtained in the Embodiment 4 (step 503), 350.5 g of the pharmaceutical composition (containing 22.5 g of ginsenoside Rg1 and Rb1, 20 g of glycyrrhizic acid and 0.005 g of jujuba cAMP) of the Example 5 of the present invention is obtained (step 504).

### Embodiment 6:

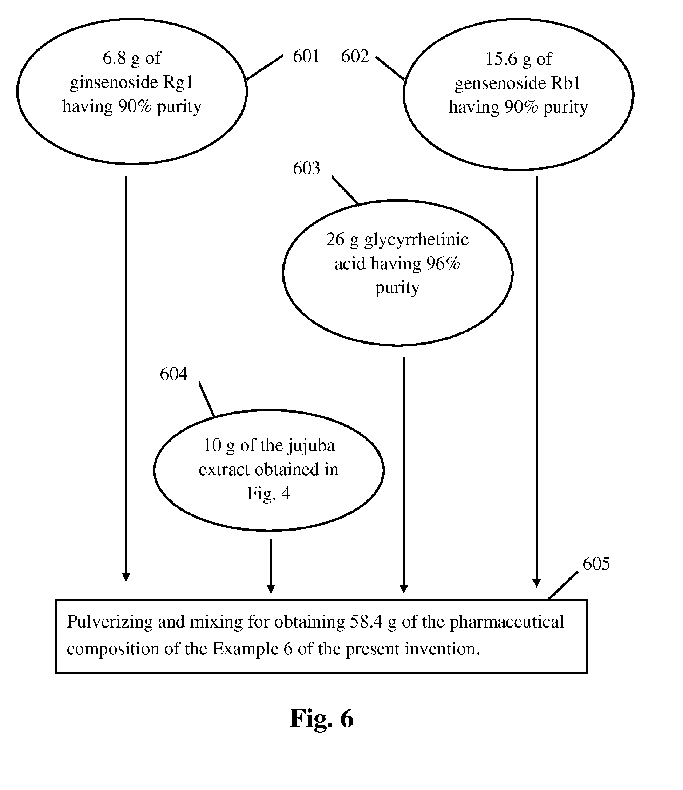

Please refer to Fig. 6, which is the flowchart showing a preparation method of a pharmaceutical composition in accordance with a sixth preferred embodiment of the present invention. In Fig. 6, after 6.8 g of the prepared ginsenoside Rg1 having 90% purity (step 601), 15.6 g of the prepared ginsenoside Rb1 having 90% purity (step 602), 26 g of glycyrrhetinic acid having 96% purity (step 603) and 10 g of the jujuba extract obtained in the Embodiment 4 (step 604) are pulverized and mixed, 58.4 g of the pharmaceutical composition (containing 20 g of ginsenoside Rg1 and Rb1, 25 g of glycyrrhetinic acid and 0.1 g of jujuba cAMP) of the Example 6 of the present invention is obtained (step 605).

### Experiment 1: The influence of Embodiment 1 in the mouse tail-hanging experiment

1.1 Experimental animals: ICR mice, male, 22.0±2 g of body weight, secondary, are provided by the Experimental Animal Science Department of Capital Medical University, Beijing.

1.2 Experiment pharmaceuticals: The pharmaceutical of Embodiment 1 is provided by Beijing Wonner Biotech. Ltd. Co., and Paroxetine (Paxil) is the product of Zhong Mei Tianjin Smith Kline pharmaceuticals Co. Ltd.

1.3 Experimental equipment: Stop watch.

1.4 Dose designs: 1. High dose of Embodiment 1 (80 mg/kg/d); 2. middle dose of Embodiment 1 (40 mg/kg/d); and 3. low dose of Embodiment 1 (20 mg/kg/d).

1.5 Experimental method and result:

1.5.1 Group division and administration of drug: The mice are grouped randomly, and 10 mice are in each group. 1. High dose of Embodiment 1 (80 mg/kg, per oral (P.O.), administered for 7 days); 2. middle dose of Embodiment 1 (40 mg/kg, P.O., administered for 7 days); 3. low dose of Embodiment 1 (20 mg/kg, P.O., administered for 7 days); 4. Paroxetine (3 mg/kg, P.O., administered for 7 days); and 5. physiological saline (P.O.). After 1 hour of the last administration of drug, the mouse tail-hanging experiment is proceeded.

1.5.2 Experimental method: The mouse's tail (1 cm to the tail end) is taped on the wood strip higher than the platform for 5 cm and hung up for 6 minutes. The time of non-movement of the mouse for the last 5 minutes is recorded.

1.5.3 Statistic calculation: The experimental data are represented as *X̅* ± *SD* , and the experimental result is calculated as analysis of variance (ANOVA) by SPSS 11.5 statistic software.

1.5.4 Experimental result: Please refer to Table 1.

**Table 1. The influence of Embodiment 1 on the time of non-movement of the mouse**

| Group | Animal number | Time of non-movement (s) |
|---|---|---|
| Physiological saline (control) | 10 | 113.22±21.18 |
| Paroxetine | 10 | 75.33±22.91* |
| High dose of Embodiment 1 | 10 | 54.67±26.38** |
| Middle dose of Embodiment 1 | 10 | 72.68±27.06* |
| Low dose of Embodiment 1 | 10 | 95.26±49.91 |

| | | |
|---|---|---|
| In comparison with the control group: *P < 0.05, and **P < 0.01. | | |

Conclusion: According to the above experiment, it can be found that the high and middle doses of Embodiment 1 of the present invention and Paroxetine all decrease the time of non-movement after the mouse's tail is hung up, significantly different in comparison with the physiological group (control). Therefore, the Embodiment 1 of the present invention having anti-experimental depression function can be extrapolated.

### Experiment 2: The influence of Embodiment 1 in the Resetpine-induced mouse body temperature decrease experiment

2.1 Experimental animals: ICR mice, male, 22.0±2 g of body weight, secondary, are provided by the Experimental Animal Science Department of Capital Medical University, Beijing.

2.2 Experiment pharmaceuticals: The pharmaceutical of Embodiment 1 is provided by Beijing Wonner Biotech. Ltd. Co., Paroxetine (Paxil) is the product of Zhong Mei Tianjin Smith Kline pharmaceuticals Co. Ltd., and Resetpine is the product of Guangdong BangMin Pharmaceutical Co., Ltd.

2.3 Experimental equipments: Electronic thermometer (Model: GM222) and stop watch.

2.4 Dose designs: 1. High dose of Embodiment 1 (80 mg/kg/d); 2. middle dose of Embodiment 1 (40 mg/kg/d); and 3. low dose of Embodiment 1 (20 mg/kg/d).

2.5 Experimental method and result:

2.5.1 Group division and administration of drug: The mice are grouped randomly, and 10 mice are in each group. 1. High dose of Embodiment 1 (80 mg/kg, P.O., administered for 7 days); 2. middle dose of Embodiment 1 (40 mg/kg, P.O., administered for 7 days); 3. low dose of Embodiment 1 (20 mg/kg, P.O., administered for 7 days); 4. Paroxetine (3 mg/kg, P.O., administered for 7 days); and 5. physiological saline (P.O.).

2.5.2 Experimental method: After 1 hour of the last administration of drug on the eighth day, the mouse's anal temperature is determined. Then 2 mg Resetpine per kilogram of the body weight is given by intraperitoneal injection. After 4 hours of injecting Resetpine, the mouse's anal temperature is determined once again. The depth and time of injecting the thermometer to the mouse's anus are identical in each temperature measurement.

2.5.3 Statistic calculation: The experimental data are represented as *X̅* ± *SD* , and the experimental result is calculated as ANOVA by SPSS 11.5 statistic software.

2.5.4 Experimental result: Please refer to Table 2.

**Table 2. The influence of Embodiment 1 on the Resetpine-induced decrease in mouse body temperature**

| Group | Animal number | Decreased temperature (°C) |
|---|---|---|
| Physiological saline (control) | 10 | 3.65±0.77 |
| Paroxetine | 10 | 2.38±0.69** |
| High dose of Embodiment 1 | 10 | 1.85±1.01** |
| Middle dose of Embodiment 1 | 10 | 2.05±1.03** |
| Low dose of Embodiment 1 | 10 | 2.35±0.69** |

| | | |
|---|---|---|
| In comparison with the control group: *P < 0.05, and **P < 0.01. | | |

Conclusion: According to the above experiment, it can be found that the high, middle and low doses of Embodiment 1 of the present invention and Paroxetine all decrease the reduced body temperature induced by Resetpine; it means that the anti-experimental depression functions of these pharmaceuticals might be related to their effects on the contents of monoamine neurotransmitter. Therefore, the Embodiment 1 of the present invention having anti-experimental depression function can be extrapolated.

### Experiment 3: The influence of Embodiment 2 in the mouse tail-hanging experiment

3.1 Experimental animals: ICR mice, male, 22.0±2 g of body weight, secondary, are provided by the Experimental Animal Science Department of Capital Medical University, Beijing.

3.2 Experiment pharmaceuticals: The pharmaceutical of Embodiment 2 is provided by Beijing Wonner Biotech. Ltd. Co., and Paroxetine (Paxil) is the product of Zhong Mei Tianjin Smith Kline pharmaceuticals Co. Ltd.

3.3 Experimental equipment: Stop watch.

3.4 Dose designs: 1. High dose of Embodiment 2 (80 mg/kg/d); 2. middle dose of Embodiment 2 (40 mg/kg/d); and 3. low dose of Embodiment 2 (20 mg/kg/d).

3.5 Experimental method and result:

3.5.1 Group division and administration of drug: The mice are grouped randomly, and 10 mice are in each group. 1. High dose of Embodiment 2 (80 mg/kg, P.O., administered for 7 days); 2. middle dose of Embodiment 2 (40 mg/kg, P.O., administered for 7 days); 3. low dose of Embodiment 2 (20 mg/kg, P.O., administered for 7 days); 4. Paroxetine (3 mg/kg, P.O., administered for 7 days); and 5. physiological saline (P.O.). After 1 hour of the last administration of drug, the mouse tail-hanging experiment is proceeded.

3.5.2 Experimental method: The mouse's tail (1 cm to the tail end) is taped on the wood strip higher than the platform for 5 cm and hung up for 6 minutes. The time of non-movement of the mouse for the last 5 minutes is recorded.

3.5.3 Statistic calculation: The experimental data are represented as *X̅* ± *SD* , and the experimental result is calculated as ANOVA by SPSS 11.5 statistic software.

3.5.4 Experimental result: Please refer to Table 3.

**Table 3. The influence of Embodiment 2 on the time of non-movement of the mouse**

| Group | Animal number | Time of non-movement (s) |
|---|---|---|
| Physiological saline (control) | 10 | 113.22±21.18 |
| Paroxetine | 10 | 75.33±22.91* |
| High dose of Embodiment 2 | 10 | 93.27±36.42 |
| Middle dose of Embodiment 2 | 10 | 76.21±28.36* |
| Low dose of Embodiment 2 | 10 | 107.79±32.56 |

| | | |
|---|---|---|
| In comparison with the control group: *P < 0.05, and **P < 0.01. | | |

Conclusion: According to the above experiment, it can be found that the middle dose of Embodiment 2 of the present invention and Paroxetine all decrease the time of non-movement after the mouse's tail is hung up, significantly different in comparison with the physiological group (control). Therefore, the Embodiment 2 of the present invention having anti-experimental depression function can be extrapolated.

### Experiment 4: The influence of Embodiment 2 in the Resetpine-induced mouse body temperature decrease experiment

4.1 Experimental animals: ICR mice, male, 22.0±2 g of body weight, secondary, are provided by the Experimental Animal Science Department of Capital Medical University, Beijing.

4.2 Experiment pharmaceuticals: The pharmaceutical of Embodiment 2 is provided by Beijing Wonner Biotech. Ltd. Co., Paroxetine (Paxil) is the product of Zhong Mei Tianjin Smith Kline pharmaceuticals Co. Ltd., and Resetpine is the product of Guangdong BangMin Pharmaceutical Co., Ltd.

4.3 Experimental equipments: Electronic thermometer (Model: GM222) and stop watch.

4.4 Dose designs: 1. High dose of Embodiment 2 (80 mg/kg/d); 2. middle dose of Embodiment 2 (40 mg/kg/d); and 3. low dose of Embodiment 2 (20 mg/kg/d).

4.5 Experimental method and result:

4.5.1 Group division and administration of drug: The mice are grouped randomly, and 10 mice are in each group. 1. High dose of Embodiment 2 (80 mg/kg, P.O., administered for 7 days); 2. middle dose of Embodiment 2 (40 mg/kg, P.O., administered for 7 days); 3. low dose of Embodiment 2 (20 mg/kg, P.O., administered for 7 days); 4. Paroxetine (3 mg/kg, P.O., administered for 7 days); and 5. physiological saline (P.O.).

4.5.2 Experimental method: After 1 hour of the last administration of drug on the eighth day, the mouse's anal temperature is determined. Then 2 mg Resetpine per kilogram of the body weight is given by intraperitoneal injection. After 4 hours of injecting Resetpine, the mouse's anal temperature is determined once again. The depth and time of injecting the thermometer to the mouse's anus are identical in each temperature measurement.

4.5.3 Statistic calculation: The experimental data are represented as *X̅* ± *SD* , and the experimental result is calculated as ANOVA by SPSS 11.5 statistic software.

4.5.4 Experimental result: Please refer to Table 4.

**Table 4. The influence of Embodiment 2 on the Resetpine-induced decrease of mouse body temperature**

| Group | Animal number | Decreased temperature (°C) |
|---|---|---|
| Physiological saline (control) | 10 | 3.65±0.77 |
| Paroxetine | 10 | 2.38±0.69** |
| High dose of Embodiment 2 | 10 | 2.93±0.74* |
| Middle dose of Embodiment 2 | 10 | 2.31±0.82** |
| Low dose of Embodiment 2 | 10 | 3.21±0.71 |

| | | |
|---|---|---|
| In comparison with the control group: *P < 0.05, and **P < 0.01. | | |

Conclusion: According to the above experiment, it can be found that the middle dose of Embodiment 2 of the present invention and Paroxetine all decrease the reduced body temperature induced by Resetpine; it means that the anti-experimental depression functions of the pharmaceuticals might be related to their effects on the contents of monoamine neurotransmitter. Therefore, the Embodiment 2 of the present invention having anti-experimental depression function can be extrapolated.

### Experiment 5: The influence of Embodiment 3 in the mouse tail-hanging experiment

5.1 Experimental animals: ICR mice, male, 22.0±2 g of body weight, secondary, are provided by the Experimental Animal Science Department of Capital Medical University, Beijing.

5.2 Experiment pharmaceuticals: The pharmaceutical of Embodiment 3 is provided by Beijing Wonner Biotech. Ltd. Co., and Paroxetine (Paxil) is the product of Zhong Mei Tianjin Smith Kline pharmaceuticals Co. Ltd.

5.3 Experimental equipment: Stop watch.

5.4 Dose designs: 1. High dose of Embodiment 3 (80 mg/kg/d); 2. middle dose of Embodiment 3 (40 mg/kg/d); and 3. low dose of Embodiment 3 (20 mg/kg/d).

5.5 Experimental method and result:

5.5.1 Group division and administration of drug: The mice are grouped randomly, and 10 mice are in each group. 1. High dose of Embodiment 3 (80 mg/kg, P.O., administered for 7 days); 2. middle dose of Embodiment 3 (40 mg/kg, P.O., administered for 7 days); 3. low dose of Embodiment 3 (20 mg/kg, P.O., administered for 7 days); 4. Paroxetine (3 mg/kg, P.O., administered for 7 days); and 5. physiological saline (P.O.). After 1 hour of the last administration of drug, the mouse tail-hanging experiment is proceeded.

5.5.2 Experimental method: The mouse's tail (1 cm to the tail end) is taped on the wood strip higher than the platform for 5 cm and hung up for 6 minutes. The time of non-movement of the mouse for the last 5 minutes is recorded.

5.5.3 Statistic calculation: The experimental data are represented as *X̅* ± *SD* , and the experimental result is calculated as ANOVA by SPSS 11.5 statistic software.

5.5.4 Experimental result: Please refer to Table 5.

**Table 5. The influence of Embodiment 3 to time of non-movement of the mouse**

| Group | Animal number | Time of non-movement (s) |
|---|---|---|
| Physiological saline (control) | 10 | 113.22±21.18 |
| Paroxetine | 10 | 75.33±22.91* |
| High dose of Embodiment 3 | 10 | 70.37±28.14* |
| Middle dose of Embodiment 3 | 10 | 76.26±23.81* |
| Low dose of Embodiment 3 | 10 | 90.40±31.32 |

| | | |
|---|---|---|
| In comparison with the control group: *P < 0.05, and **P < 0.01. | | |

Conclusion: According to the above experiment, it can be found that the high and middle doses of Embodiment 3 of the present invention and Paroxetine can all decrease the time of non-movement after the mouse's tail is hung up, significantly different from the physiological group (control). Therefore, the Embodiment 3 of the present invention having anti-experimental depression function can be extrapolated.

### Experiment 6: The influence of Embodiment 3 in the Resetpine-induced mouse body temperature decrease experiment

6.1 Experimental animals: ICR mice, male, 22.0±2 g of body weight, secondary, are provided by the Experimental Animal Science Department of Capital Medical University, Beijing.

6.2 Experiment pharmaceuticals: The pharmaceutical of Embodiment 3 is provided by Beijing Wonner Biotech. Ltd. Co., Paroxetine (Paxil) is the product of Zhong Mei Tianjin Smith Kline pharmaceuticals Co. Ltd., and Resetpine is the product of Guangdong BangMin Pharmaceutical Co., Ltd.

6.3 Experimental equipments: Electronic thermometer (Model: GM222) and stop watch.

6.4 Dose designs: 1. High dose of Embodiment 3 (80 mg/kg/d); 2. middle dose of Embodiment 3 (40 mg/kg/d); and 3. low dose of Embodiment 3 (20 mg/kg/d).

6.5 Experimental method and result:

6.5.1 Group division and administration of drug: The mice are grouped randomly, and 10 mice are in each group. 1. High dose of Embodiment 3 (80 mg/kg, P.O., administered for 7 days); 2. middle dose of Embodiment 3 (40 mg/kg, P.O., administered for 7 days); 3. low dose of Embodiment 3 (20 mg/kg, P.O., administered for 7 days); 4. Paroxetine (3 mg/kg, P.O., administered for 7 days); and 5. physiological saline (P.O.).

6.5.2 Experimental method: After 1 hour of the last administration of drug on the eighth day, the mouse's anal temperature is determined. Then 2 mg Resetpine per kilogram of the body weight is given by intraperitoneal injection. After 4 hours of injecting Resetpine, the mouse's anal temperature is determined once again. The depth and time of injecting the thermometer to the mouse's anus are identical in each temperature measurement.

6.5.3 Statistic calculation: The experimental data are represented as *X̅* ± *SD* , and the experimental result is calculated as ANOVA by SPSS 11.5 statistic software.

6.5.4 Experimental result: Please refer to Table 6.

**Table 6. The influence of Embodiment 3 on the decreased mouse body temperature induced by Resetpine**

| Group | Animal number | Decreased temperature (°C) |
|---|---|---|
| Physiological saline (control) | 10 | 3.65±0.77 |
| Paroxetine | 10 | 2.38±0.69** |
| High dose of Embodiment 3 | 10 | 2.18±0.92** |
| Middle dose of Embodiment 3 | 10 | 2.36±0.83** |
| Low dose of Embodiment 3 | 10 | 2.97±0.67* |

| | | |
|---|---|---|
| In comparison with the control group: *P < 0.05, and **P < 0.01. | | |

Conclusion: According to the above experiment, it can be found that the high, middle and low doses of Embodiment 3 of the present invention and Paroxetine can all decrease the reduced body temperature induced by Resetpine, and it means that the anti-experimental depression functions of the pharmaceuticals might be related to their effects on the contents of monoamine neurotransmitter. Therefore, the Embodiment 3 of the present invention having anti-experimental depression function can be extrapolated.

### Experiment 7: The influence of Embodiment 4 in the mouse olfactory bulb lesion experiment

7.1 Experimental animals:

Olfactory bulb lesion model: Health Wistar male rats, secondary, 330±20 g of body weight, are purchased from Beijing Vital River Experimental Animal Technology Ltd. Co. (The quality certificate number: SCXK (JING) 2002-2003).

7.2 Reagents and pharmaceuticals: The pharmaceutical of Embodiment 4 is provided by Beijing Wonner Biotech Ltd. Co. (Lot: 060313), and Paroxetine is the product of Zhong Mei Tianjin Smith Kline pharmaceuticals Co. Ltd. (Lot: 04050011). The above pharmaceuticals are prepared with 0.5% of sodium carboxymethylcellulose (CMC-Na) for feeding into the stomach. Benzylpenicillin sodium for injection is the product of North China Pharmaceutical Huasheng Co. Ltd. (Lot: S0511204), and norepinephrine (NE) and 5-hydrotryptamine (5-HT) standards are the products of Sigma Co.. Other reagents are all marketed.

7.3 Equipments: Self-made open field activity box, step through box, rat stereotaxic instrument, high performance liquid chromatography (HPLC), and 10-tube γ radiation immunity arithmometer (Mode: DFM-96).

7.4 Experimental methods:

7.4.1 Group division and administration of drug: The rats are grouped randomly into 6 groups. 1. False therapy group; 2. model group (control); 3. high dose of Embodiment 4 (60 mg/kg/d); 4. middle dose of Embodiment 4 (30 mg/kg/d); 5. low dose of Embodiment 4 (15 mg/kg/d); and 6. Paroxetine (2 mg/kg/d). The test drugs and the positive drug are prepared with 0.5% of CMC-Na for feeding into the stomach once every day.

7.4.2 Preparation method of Model: The rat is anesthetized by chloral hydrate. After anesthesia, the linea median of the rat's fontanel is carved from 1 cm prior to the anterior fontanel to 1 cm behind the anterior fontanel, and the ossa cranii is exposed. The skull windows having 2 mm of diameter are opened from 8 mm prior to the anterior fontanel and from 2 mm of two sides of the linea median. The specially made electric soldering iron is inserted perpendicularly into the skull for 2 seconds, and the olfactory bulb is destroyed. The hemostatic sponge is filled into the skull windows and the skin is sewn. After the therapy, 40,000 unit of benzylpenicillin sodium per kilogram of the body weight is given by intraperitoneal injection for every four days, and the tested pharmaceutical is taken continuously for 24 days.

7.5 Observation indexes:

7.5.1 Open field activity experiment: The open field activity box (1 m x 1 m x 0.4 m) is constructed by the light blue plywood and the aluminum alloy frame. The box bottom is separated into 25 grids (20 cm x 20 cm for each grid), the circumference is the peripheral grids (16 grids), and others are the central grids (9 grids). The rat is placed in the center of the central grids, and the rat's cross-grid number (the number of crossing into the neighboring gird more than 3 claws) and rat's standing number (two forelimbs leaving the ground for more than 1 cm) are calculated/observed within 3 minutes.

7.5.2 Passive avoidance experiment (Step-through test): The step through box is configured by the light chamber and the dark chamber, and a channel is linked between the bright chamber and the dark chamber for mouse entrance and exit. The grille of the dark chamber is connected to the electric shock equipment, and a mobile plate is set there between. If the rat enters into the dark chamber, the rat will be electrically shocked. During training, the rat is placed in the light chamber and is back in the hole for adaptation for 5 minutes. Then the plate is removed and the rat is observed for another 5 minutes. The time that the rat enters into for the first time is recorded (electric-shocking latent period), and the time thereof is the learning record. After 24 hours, the test is repeated. The plate is removed and the dark chamber is electrified for 5 minutes so as to observe the time that the rat enters into the dark chamber for the first time. This is thereof the memory record.

7.6 Statistic calculation: The experimental data are represented as *X̅* ± *SD ,* and the experimental result is calculated as ANOVA by SPSS 11.5 statistic software.

7.7 Experimental results:

7.7.1 The result of the open field activity experiment: Please refer to Table 7.

**Table 7. The result of the open field activity experiment of the rat olfactory bulb lesion model**

| Group | Animal number | Horizontal movement (cross-grid number) | Vertical movement (standing number) |
|---|---|---|---|
| High dose of Embodiment 4 | 11 | 49.18±27.68** | 10.91±6.91** |
| Middle dose of Embodiment 4 | 11 | 54.55±23.01 | 13.45±5.72* |
| Low dose of Embodiment 4 | 11 | 61.82±21.43 | 15.18±4.47 |
| Paroxetine | 11 | 55.36±25.96* | 14.36±5.55 |
| Model group | 11 | 79.55±24.33 | 19.09±8.53 |
| False therapy group | 11 | 45.36±26.84** | 10.45±6.19** |

| | | | |
|---|---|---|---|
| In comparison with the control group: *P < 0.05, and **P < 0.01. | | | |

7.7.2 The result of the step through test: Please refer to Table 8.

**Table 8. The result of the step through test of the rat olfactory bulb lesion model**

| Group | Animal number | Latent period of the 1st day (s) | Latent period of the 2nd day (s) |
|---|---|---|---|
| High dose of Embodiment 4 | 11 | 187.00±87.59* | 289.82±28.59* |
| Middle dose of Embodiment 4 | 11 | 191.71±72.95* | 288.82±37.09* |
| Low dose of Embodiment 4 | 11 | 152.44±76.81 | 271.18±38.61 |
| Paroxetine | 11 | 181.87±90.54* | 265.00±65.39 |
| Model group | 11 | 111.21±82.93 | 236.88±74.17 |
| False therapy group | 11 | 211.46±82.10** | 292.82±14.37* |

| | | | |
|---|---|---|---|
| In comparison with the control group: *P < 0.05, and **P < 0.01. | | | |

Conclusion: The result of Experiment 7 shows that the high dose of Embodiment 4 can obviously reduce the increase of the rat's horizontal and vertical movements caused by the olfactory bulb lesion, and the middle dose of Embodiment 4 also has obvious improving function on the increase of the vertical movement in the rat olfactory bulb lesion model. In addition, the high and middle doses of Embodiment 4 have obvious improving effects on the decreases of the rat study and memory functions caused by the olfactory bulb lesion.

### Experiment 8: The influence of Embodiment 4 examined in the rat unpredictable long-term stimulus experiment

8.1 Experimental animals:

Unpredictable long-term stimulus model: Health Wistar male rats, secondary, 240 ∼ 270 g of body weight, are purchased from Beijing Vital River Experimental Animal Technology Ltd. Co. (The quality certificate number: SCXK (JING) 2002-2003).

8.2 Reagents and pharmaceuticals: The pharmaceutical of Embodiment 4 is provided by Beijing Wonner Biotech Ltd. Co. (Lot: 060313), and Paroxetine is the product of Zhong Mei Tianjin Smith Kline pharmaceuticals Co. Ltd. (Lot: 04050011). The above pharmaceuticals are prepared with 0.5% of sodium carboxymethylcellulose (CMC-Na) for feeding into the stomach. Benzylpenicillin sodium for injection is the product of North China Pharmaceutical Huasheng Co. Ltd. (Lot: S0511204), and norepinephrine (NE) and 5-hydrotryptamine (5-HT) standards are the products of Sigma Co. Other reagents are all marketed.

8.3 Equipments: Self-made open field activity box, step through box, rat stereotaxic instrument, high performance liquid chromatography (HPLC), and 10-tube γ radiation immunity arithmometer (Mode: DFM-96).

8.4 Experimental methods:

8.4.1 Group division and administration of drug: The rats are grouped randomly into 6 groups. 1. False therapy group; 2. model group (control); 3. high dose of Embodiment 4 (60 mg/kg/d); 4. middle dose of Embodiment 4 (30 mg/kg/d); 5. low dose of Embodiment 4 (15 mg/kg/d); and 6. Paroxetine (2 mg/kg/d). The test drugs and the positive control drug are prepared with 0.5% of CMC-Na for feeding into the stomach once every day.

8.4.2 Preparation method of Model:

Unpredictable long-term stimulus model: The rats in the control group eat and drink normally, and there is no stimulus performed. In other 5 groups, one rat is fed in each cage, and the rat is subject to a 24-day unpredictable stress/stimulus, including 24-hour abstinence for 3 times, 24-hour without drinking for 3 times, 24-hour wet bedding for 3 times (200 ml of water is added in the rat cage), overnight illumination for 3 times, swimming at 4°C for 5 minutes for 3 times, heating at 45°C in the oven for 5 minutes for 3 times, clipping the rat tail for 1 minute for 3 times, and 30-minute high-speed horizontal shake for 3 times. One stimulus is performed randomly every day and a total of 24 days. Each stimulus cannot be performed continuously. The pharmaceutical is fed into the rat's stomach once every day for a total of 24 days.

8.5 Observation indexes:

8.5.1 Open field activity experiment: Same as above.

8.5.2 Passive avoidance experiment: Same as above.

8.5.3 Rat swimming by compulsion: This experiment is proceeded for two days after the last administration of drug. On the first day, the experiment is pre-performed for 15 minutes. The water temperature in the glass tank is 25°C, and the depth of water is 25 cm. After 24 hours, the formal experiment is proceeded. After 1 hour of administration of drug, the rat is placed in the tank, and the time of non-movement of the rat for the last 5 minutes is recorded.

8.5.4 Body weight measurement: The increasing values before and after each animal experiment are compared.

8.5.5 Volume test of drinking sucrose: The sucrose-intake volume of the rats are compared. The rats in each group drink 1% of the sucrose for 1 hour. The drinking volumes are determined before the stimulus and after 3 weeks of the stimulus. After the rat is in abstinence and water for 14 hours, 1% of the sucrose is placed in the cage and substituted for the original drinking water. The differences between the bottle weight before and after the rat drinking sucrose for 1 hour are measured and recorded, and the sucrose-drinking volume for each time is calculated. The difference of the sucrose-intake volume for each time is compared.

8.5.6 HPLC-electrochemical test: The amounts of norepinephrine and 5-hydrotryptamine in the rat cerebral cortex are measured.

8.6 Statistic calculation: The experimental data are represented as *X̅* ± *SD* , and the experimental result is calculated as ANOVA by SPSS 11.5 statistic software.

8.7 Experimental results:

8.7.1 Volume test of drinking sucrose: please refer to Table 9.

**Table 9. The sucrose-intake volume of the rat in the unpredictable long-term stimulus model**

| Group | Animal number | Sucrose-intake volume (g) |
|---|---|---|
| High dose of Embodiment 4 | 13 | 22.55±5.03 |
| Middle dose of Embodiment 4 | 13 | 26.53±4.99** |
| Low dose of Embodiment 4 | 13 | 26.97±6.93** |
| Paroxetine | 13 | 26.29±4.87** |
| Model | 13 | 19.42±4.25 |
| False therapy group | 13 | 29.41±3.83** |

| | | |
|---|---|---|
| In comparison with the control group: *P < 0.05, and **P < 0.01. | | |

8.7.2 The result of the increasing rat body weight: Please refer to Table 10.

**Table 10. The increase in rat body weight in the unpredictable long-term stimulus model**

| Group | Animal number | Increasing body weight (g) |
|---|---|---|
| High dose of Embodiment 4 | 13 | 86.08±10.84 |
| Middle dose of Embodiment 4 | 13 | 96.00±11.05** |
| Low dose of Embodiment 4 | 13 | 95.15±15.46** |
| Paroxetine | 13 | 96.85±9.30** |
| Model group (control) | 13 | 84.92±12.61 |
| False therapy group | 13 | 120.54±10.60** |

| | | |
|---|---|---|
| In comparison with the control group: *P < 0.05, and **P < 0.01. | | |

8.7.3 The result of the time of non-movement in the rat swimming by compulsion experiment: Please refer to Table 11.

**Table 11. The time of non-movement of the rat swimming by compulsion experiment in the unpredictable long-term stimulus model**

| Group | Animal number | Time of non-movement (s) |
|---|---|---|
| High dose of Embodiment 4 | 13 | 23.28±18.05** |
| Middle dose of Embodiment 4 | 13 | 18.95±12.55** |
| Low dose of Embodiment 4 | 13 | 25.20±13.60* |
| Paroxetine | 13 | 31.38±19.59 |
| Model group (control) | 13 | 39.95±17.46 |
| False therapy group | 13 | 26.96±12.76* |

| | | |
|---|---|---|
| In comparison with the control group: *P < 0.05, and **P < 0.01. | | |

8.7.4 The result of the rat open field activity experiment: Please refer to Table 12.

**Table 12. The result of the rat open filed activity experiment in the unpredictable long-term stimulus model**

| Group | Animal number | Horizontal movement (cross-grid number) | Vertical movement (standing number) |
|---|---|---|---|
| High dose of Embodiment 4 | 14 | 58.31±15.35* | 16.54±4.24** |
| Middle dose of Embodiment 4 | 14 | 49.15±14.26 | 13.54±4.48 |
| Low dose of Embodiment 4 | 14 | 52.62±21.83 | 16.15±7.32* |
| Paroxetine | 14 | 61.85±21.68** | 14.69±4.2 |
| Model group (control) | 14 | 39.54±16.31 | 11.46±3.26 |
| False therapy group | 14 | 64.00±11.97** | 16.85±3.18** |

| | | | |
|---|---|---|---|
| In comparison with the control group: *P < 0.05, and **P < 0.01. | | | |

8.7.5 The result of the rat step-through test: Please refer to Table 13.

**Table 13. The result of the rat step-through test in the unpredictable long-term stimulus model**

| Group | Animal number | Latent period of first day (s) | Latent period of second day (s) |
|---|---|---|---|
| High dose of Embodiment 4 | 13 | 65.43±31.44 | 259.22±56.51 |
| Middle dose of Embodiment 4 | 13 | 58.18±18.0 | 212.76±77.27 |
| Low dose of Embodiment 4 | 13 | 75.98±32.22* | 204.85±94.99 |
| Paroxetine | 13 | 75.75±46.52* | 257.46±66.92 |
| Model group (control) | 13 | 50.01±15.7 | 189.25±111.99 |
| False therapy group | 13 | 80.00±39.17* | 263.38±59.68 |

| | | | |
|---|---|---|---|
| In comparison with the control group: *P < 0.05, and **P < 0.01. | | | |

8.7.6 The result of NE and 5-HT contents in the rat cerebral cortex: Please refer to Table 14.

**Table 14. The result of NE and 5-HT contents in the rat cerebral cortex in the unpredictable long-term stimulus model**

| Group | Animal number | 5-HT (nmol/L brain tissue extract) | NE (nmol/L brain tissue extract) |
|---|---|---|---|
| High dose of Embodiment 4 | 12 | 230.4157±47.78554* | 269.5409±58.86389** |
| Middle dose of Embodiment 4 | 12 | 303.4418±70.31711** | 227.2976±28.95101** |
| Low dose of Embodiment 4 | 12 | 332.7343±76.25168** | 201.8688±29.80775** |
| Paroxetine | 12 | 227.0637±46.53838* | 220.5419±38.31681** |
| Model group (control) | 12 | 179.3866±20.49374 | 57.6671±77.66958 |
| False therapy group | 12 | 228.1478±28.40397* | 132.8598±20.84756** |

| | | | |
|---|---|---|---|
| In comparison with the control group: *P < 0.05, and **P < 0.01. | | | |

Conclusion: The results of Experiment 8 are shown as follows. The middle and low doses of Embodiment 4 can obviously improve the decreased sucrose-drinking volume and the decreased body weight in response to the unpredictable long-term stress/stimulus. The high, middle and low doses of Embodiment 4 can obviously increase the time of non-movement of the rat swimming by compulsion. The high dose of Embodiment 4 can obviously improve the decreased rat horizontal and vertical movements caused by the unpredictable long-term stress/stimulus. The low dose of Embodiment 4 also has obviously improved function on the decreased rat vertical movement caused by the unpredictable long-term stress/stimulus. The low dose of Embodiment 4 has improved function on the decreased rat learning ability caused by the unpredictable long-term stress/stimulus. The high, middle and low doses of Embodiment 4 all can obviously increase NE and 5-HT contents in the rat cerebral cortex.

### Experiment 9: The influence of Embodiment 5 in the mouse tail-hanging experiment

9.1 Experiment pharmaceuticals: The pharmaceutical of Embodiment 5 is provided by Beijing Wonner Biotech. Ltd. Co. (pilot magnification product), and Paroxetine is the product of Zhong Mei Tianjin Smith Kline pharmaceuticals Co. Ltd. (Lot: 05070384). Above-mentioned pharmaceuticals are prepared with physiological saline for feeding into the stomach.

9.2 Experimental animals: ICR mice, male, 20.0±1 g of body weight, secondary, are provided by the Experimental Animal Science Department of Capital Medical University, Beijing. The quality certificate number of mice is SCXK (JING) 2006-2008.

9.3 Experimental equipment: Stop watch.

9.4 Method: Seventy (70) mice are randomly grouped into 5 groups: normal saline (NS) group, Paroxetine (3 mg/kg/d), high dose of Embodiment 5 (80 mg/kg/d), middle dose of Embodiment 5 (40 mg/kg/d), and low dose of Embodiment 5 (20 mg/kg/d). The pharmaceuticals are fed into mouse's stomach once every day. After 1 hour of the last administration of drug on the eighth day, the mouse's tail (1 cm to the tail end) is taped on the horizontal support in an opened box, and the mouse represents the reversed hung status. The mouse head is away from the bottom of the opened box for about 10 cm, and the mouse is hung up for 6 minutes. The time of non-movement of the mouse for the last 5 minutes is recorded.

9.5 Statistic calculation: The experimental data are represented as *X̅* ± *SD* , and the experimental result is calculated as one-way ANOVA by SPSS 11.5 statistic software.

9.6 Experimental result: The result of the time of non-movement in the mouse tail-hanging experiment: Please refer to Table 15.

**Table 15. The influence of Embodiment 5 on the accumulative time of non-movement in the mouse tail-hanging experiment**

| Group | Animal number | Dose (mg/kg/d) | Time of non-movement (s) |
|---|---|---|---|
| High dose of Embodiment 5 | 14 | 80 | 64.75±42.22** |
| Middle dose of Embodiment 5 | 14 | 40 | 55.41±33.83** |
| Low dose of Embodiment 5 | 14 | 20 | 62.75±26.61** |
| Paroxetine | 14 | 3 | 53.27±20.02** |
| NS group | 14 | | 113.59±36.11 |

| | | | |
|---|---|---|---|
| In comparison with the NS group: *P < 0.05, and **P < 0.01. | | | |

Conclusion: The research result shows that the high, middle and low doses of Embodiment 5 and the clinical effective anti-depression pharmaceutical, Paroxetine, all can obviously decrease the accumulative time of non-movement in the mouse tail-hanging experiment. It indicates that Embodiment 5 has specific anti-experimental depression function.

### Experiment 10: The influence of Embodiment 5 in the mouse swimming by compulsion experiment

10.1 Experiment pharmaceuticals: The pharmaceutical of Embodiment 5 is provided by Beijing Wonner Biotech. Ltd. Co. (pilot magnification product), and Paroxetine is the product of Zhong Mei Tianjin Smith Kline pharmaceuticals Co. Ltd. (Lot: 05070384). Above-mentioned pharmaceuticals are prepared with physiological saline for feeding into the stomach.

10.2 Experimental animals: ICR mice, male, 20.0±1 g of body weight, secondary, are provided by the Experimental Animal Science Department of Capital Medical University, Beijing. The quality certificate number of mice is SCXK (JING) 2006-2008.

10.3 Experimental equipment: Stop watch.

10.4 Experimental method: The mouse group and the administration of drugs are like the mouse tail-hanging experiment. The experiment is proceeded in tested mouse in each group after 1 hour of the administration of drug. The mouse is trained to swim for 15 minutes before the experiment and on the eighth day. After 24 hours, the experiment is performed. The mouse is placed in the glass tank having 10 cm of the water depth, 14 cm of the diameter and at 25°C of the water temperature. The accumulative time of non-movement of the mouse in the water for the last 5 minutes is recorded.

10.5 Statistic calculation: The experimental data are represented as *X̅* ± *SD* , and the experimental result is calculated as one-way ANOVA by SPSS 11.5 statistic software.

10.6 Experiment result: The result of the mouse swimming by compulsion: Please refer to Table 16.

**Table 16. The influence of Embodiment 5 in the mouse swimming by compulsion experiment**

| Group | Animal number | Dose (mg/kg/d) | Time of non-movement (s) |
|---|---|---|---|
| High dose of Embodiment 5 | 14 | 80 | 88.35±51.64* |
| Middle dose of Embodiment 5 | 14 | 40 | 65.87±38.96** |
| Low dose of Embodiment 5 | 14 | 20 | 88.12±38.57* |
| Paroxetine | 14 | 3 | 57.80±38.07** |
| NS group | 14 | | 132.47±40.64 |

| | | | |
|---|---|---|---|
| In comparison with the NS group: *P < 0.05, and **P < 0.01. | | | |

Conclusion: The research result shows that the high, middle and low doses of Embodiment 5 and the clinical effective anti-depression pharmaceutical, Paroxetine, can all obviously decrease the accumulative time of non-movement in the mouse swimming by compulsion test. It indicates that the Embodiment 5 has specific anti-experimental depression function.

### Experiment 11:

Nine (9) kg of the remained ginseng debris, 7 kg of the remained liquorice debris and 0.9 kg of the jujuba debris from the Embodiments 1 and 4 extraction procedures are collected, dried, pulverized, and mixed well for obtaining the debris mixture containing the trace amount of ginsenoside Rg1 and Rb1, glycyrrhizic acid and jujuba cAMP. The control experiment of examining the influence of the debris in the mouse tail-hanging test is proceeded.

11.1 Experimental animals: ICR mice, male, 22.0±2 g of body weight, secondary, are provided by the Experimental Animal Science Department of Capital Medical University, Beijing.

11.2 Experiment pharmaceuticals: The debris mixture is provided by Beijing Wonner Biotech. Ltd. Co., and Paroxetine (Paxil) is the product of Zhong Mei Tianjin Smith Kline pharmaceuticals Co. Ltd.

11.3 Experimental equipment: Stop watch.

11.4 Dose designs: 1. High dose of debris mixture (160 mg/kg/d); 2. middle dose of debris mixture (80 mg/kg/d); and 3. low dose of debris mixture (40 mg/kg/d).

11.5 Experimental method and result:

11.5.1 Group division and administration of drug: The mice are grouped randomly with 10 mice in each group. 1. High dose of debris mixture (160 mg/kg, P.O., administered for 7 days); 2. middle dose of debris mixture (80 mg/kg, P.O., administered for 7 days); 3. low dose of debris mixture (40 mg/kg, P.O., administered for 7 days); 4. Paroxetine (3 mg/kg, P.O., administered for 7 days); and 5. physiological saline (P.O.). After 1 hour of the last administration of drug, the mouse tail-hanging experiment is proceeded.

11.5.2 Experimental method: The mouse's tail (near to the tail end for 1 cm) is taped on the wood strip higher than the platform for 5 cm and hung up for 6 minutes. The time of non-movement of the mouse for the last 5 minutes is recorded.

11.5.3 Statistic calculation: The experimental data are represented as *X̅* ± *SD* , and the experimental result is calculated as ANOVA by SPSS 11.5 statistic software.

11.5.4 Experimental result: Please refer to Table 17.

**Table 17. The influence of debris mixture on the time of non-movement of the mouse**

| Group | Animal number | Time of non-movement (s) |
|---|---|---|
| Physiological saline (control) | 10 | 82.03±43.01 |
| Paroxetine | 10 | 38.37±20.76* |
| High dose of debris mixture | 10 | 76.91±31.09 |
| Middle dose of debris mixture | 10 | 78.89±48.18 |
| Low dose of debris mixture | 10 | 81.31±58.68 |

| | | |
|---|---|---|
| In comparison with the control group: *P < 0.05, and **P < 0.01. | | |

Conclusion: According to the above experiment, it can be found that although the high, middle and low doses of the debris mixture can shorten the time of non-movement of the mouse with tail hanging, these differences are not statistically significant in comparison with the physiological saline group (control). Therefore, the conclusion that the debris mixture lacks anti-experimental depression function can be extrapolated.

The application scopes of the oral pharmaceutical of the present invention for treating depression lie in that:

1. the described oral pharmaceutical of the present invention for treating depression can include the pharmacologically acceptable additives;

2. the described oral pharmaceutical of the present invention for treating depression can be manufactured as the known dosage forms, such as powder, capsule, tablet, etc.; and

3. the described oral pharmaceutical of the present invention for treating depression can be manufactured as the health food for treating depression.

## Claims

1. A pharmaceutical composition with a multi-target post-receptor mechanism for treating a depression, comprising:
a ginsenoside having an Rg1 and an Rb1; and
a glycyrrhizically related acid being one selected from a group consisting of a glycyrrhizic acid, a glycyrrhetinic acid and a combination thereof.

2. The pharmaceutical composition according to claim 1 comprising 2 ∼ 26 parts by weight of the ginsenoside having the Rg1 and the Rb1 and 3 ∼ 48 parts by weight of the glycyrrhizically related acid.

3. The pharmaceutical composition according to claim 1 comprising 4 ∼ 13 parts by weight of the ginsenoside having the Rg1 and the Rb1 and 5 ∼ 16 parts by weight of the glycyrrhizically related acid.

4. The pharmaceutical composition according to claim 1, wherein the ginsenoside is a ginseng extract comprising ginsenosides Rg1 and Rb1.

5. The pharmaceutical composition according to claim 1 comprising at least one of a pharmacologically acceptable carrier and an additive.

6. The pharmaceutical composition according to claim 1 having a dosage form being one selected from a group consisting of a tablet, a capsule, a powder, a pill, a dust, a solution, a microcapsule, a suspension, an emulsion, a particle, a dropping pill and a roll.

7. The pharmaceutical composition according to claim 1 being manufactured as one of a health food and a nutrient supplement.

8. A multi-target post-receptor pharmaceutical composition for treating a depression, comprising:
a ginsenoside having an Rg1 and an Rb1;
a glycyrrhizically related acid being one selected from a group consisting of a glycyrrhizic acid, a glycyrrhetinic acid and a combination thereof; and
a jujuba cyclic adenosine monophosphate (jujuba cAMP).

9. The pharmaceutical composition according to claim 8 comprising 2 ∼ 26 parts by weight of the ginsenoside having the Rg1 and the Rb1, 3 ∼ 48 parts by weight of the glycyrrhizically related acid and 0.002 - 0.5 parts by weight of the jujuba cAMP.

10. The pharmaceutical composition according to claim 8 comprising 4 ∼ 13 parts by weight of the ginsenoside having the Rg1 and the Rb1, 5 ∼ 16 parts by weight of the glycyrrhizically related acid and 0.01 ∼ 0.1 parts by weight of the jujuba cAMP.

11. The pharmaceutical composition according to claim 8, wherein the ginsenoside is a ginseng extract comprising ginsenosides Rg1 and Rb1, the liquorice is a liquorice extract comprising the glycyrrhizic acid, and the jujuba cAMP is a jujuba extract comprising the jujuba cAMP.

12. The pharmaceutical composition according to claim 8, wherein a material comprising the jujuba cAMP is a second extract obtained by extracting a liquorice to obtain a first extract firsly and then purifying the first extract, wherein a jujuba cAMP concentration of the second extract is higher than that of the first extract.

13. The pharmaceutical composition according to claim 8 comprising at least one of a pharmacologically acceptable carrier and an additive.

14. The pharmaceutical composition according to claim 8 having a dosage form being one selected from a group consisting of a tablet, a capsule, a powder, a pill, a dust, a solution, a microcapsule, a suspension, an emulsion, a particle, a dropping pill and a roll.

15. The pharmaceutical composition according to claim 8 being manufactured as one of a health food and a nutrient supplement.

16. A preparation method of a pharmaceutical composition with a multi-target post-receptor mechanism for treating a depression, the pharmaceutical composition comprising a jujuba cyclic monophosphate (jujuba cAMP) as a raw material, the preparation method comprising steps of:
(a) extracting a jujuba for obtaining a first extract; and
(b) purifying the first extract to obtain a second extract,
wherein a jujuba cAMP concentration of the second extract is higher than that of the first exttract.

17. The preparation method according to claim 16, wherein the step (b) is processed by chromatographing the jujuba cAMP in the first extract with a macroporous resin bound with an aldehyde group.

18. The preparation method according to claim 17, wherein the step (b) is processed by chromatographing the jujuba cAMP in the first extract with a OU-2 macroporous resin bound with the aldehyde group.

19. The preparation method according to claim 18, wherein the step (b) further is processed by chromatographing the jujuba cAMP in the first extract with a ME-2 is macroporous resin bound with the aldehyde group.
